# EUROPÄISCHE PATENTSCHRIFT

(11) **EP 4 210 570 B1**
(45) Veröffentlichungstag und Bekanntmachung des Hinweises auf die Patenterteilung: **19.03.2025**
(21) Anmeldenummer: 21731907.8
(22) Anmeldetag: 26.05.2021
(51) Int. Cl.: A61B 5/083, A61M 16/00, A61M 16/08

(54) **VERFAHREN UND VORRICHTUNG ZUR MESSUNG DES GEHALTS ZUMINDEST EINES GASES IN AUSATEMLUFT**
METHOD AND DEVICE FOR MEASURING THE CONTENT OF AT LEAST ONE GAS IN EXHALED AIR
PROCÉDÉ ET DISPOSITIF DE MESURE DE LA TENEUR EN AU MOINS UN GAZ DANS L'AIR EXPIRÉ

(30) Priorität: 10.09.2020 DE 102020123623
(43) Veröffentlichungstag der Anmeldung: 19.07.2023
(73) Patentinhaber: Weinmann Emergency Medical Technology GmbH + Co. KG, 22525 Hamburg (DE)
(72) Erfinder: POLERT, Annika, 22850 Norderstedt (DE); KREUZER, Johannes, 21079 Hamburg (DE); NEUHAUS, Christian, 25451 Quickborn (DE)
(74) Vertreter: Klickow & Wetzel PartGmbB
(86) Internationale Anmeldenummer: PCT/DE2021/100455
(87) Internationale Veröffentlichungsnummer: WO 2022/053094

(56) Entgegenhaltungen:
- DE-A1- 102008 022 761
- US-A1- 2006 201 507
- US-A1- 2013 165 806

## Beschreibung

Die Erfindung betrifft eine Vorrichtung zur Messung des Gehalts zumindest eines Gases in Ausatemluft insbesondere zur Verwendung mit einem Beatmungsgerät.

Darüber hinaus betrifft die Erfindung ein Verfahren zur Messung des Gehalts zumindest eines Gases in Ausatemluft.

Weiterhin betrifft die Erfindung eine Vorrichtung zur Beatmung im Sinne eines Beatmungsgeräts, insbesondere eines Notfallbeatmungsgeräts, aufweisend eine Vorrichtung zur Messung des Gehalts zumindest eines Gases in Ausatemluft.

Im Zuge der zumindest teilweise manuellen oder der maschinellen Beatmung von Patienten mit Luft ist die Bestimmung verschiedener Gasgehalte sowohl in der dem Patienten zugeführten Luft als auch in der Ausatemluft von Interesse.

Beispielsweise erfolgt die Bestimmung des Kohlendioxid-Gehalts (CO2) oder Sauerstoffgehalts (O2) unter Verwendung von geeigneten Sensoren.

Je nach Anwendungsfall ist dabei eine zeitlich hochaufgelöste Messung des jeweiligen Gases erforderlich.

Der Atemzyklus eines Menschen und anderer entsprechend atmender Lebewesen setzt sich aus der Einatmung (Inspiration) und der Ausatmung (Exspiration) zusammen. Wird Umgebungsluft eingeatmet, so beträgt die inspiratorische Sauerstoffkonzentration (FiO2) etwa 20,9 Vol.-%. Am Ende der Ausatmung verbleibt nach dem Gasaustausch zwischen Lunge und Herz-Kreislaufsystem eine endtidale Sauerstoffkonzentration (etO2) von etwa 16 Vol.-% in der Ausatemluft. Bei der Einatmung liegt der CO2-Gehalt (FiCO2) der Atemluft etwa bei 0 Vol.-% (0,04 Vol.-% CO2 in der Umgebungsluft), während dieser bei der Ausatmung deutlich ansteigt, sodass eine endtidale CO2-Konzentration (etCO2) von ca. 4,5 Vol.-% messbar ist.

In der Medizin ist es beispielsweise üblich, den Verlauf des CO2-Gehalts in der Ausatemluft während der Atmung eines Patienten zu bestimmen. Am Ende der Ausatmung ist die endtidale CO2-Konzentration (etCO2) bestimmbar, sofern eine entsprechend schnelle und damit zeitlich entsprechend hochauflösende CO2-Sensortechnologie eingesetzt wird. Dadurch ist das Konzentrationsplateau am Ende der Ausatmung bestimmbar.

Zur Messung des CO2-Gehaltes in der Atemluft gibt es gemäß dem Stand der Technik grundsätzlich zwei Verfahren, die im Zusammenhang mit der Beatmung von Patienten Anwendung finden. Das Atemgas strömt je nach Atemphase durch ein Schlauchsystem der Vorrichtung zur Beatmung zum Patienten hin oder von diesem weg. Im Hauptstromverfahren wird das komplette Atemgas des Patienten direkt in bzw. an diesem Schlauchsystem und somit im Hauptstrom des Atemgases analysiert, wohingegen im Nebenstromverfahren ein Probengas aus dem Hauptstrom abgesaugt und analysiert wird. Die Absaugung des Probengases erfolgt dabei in der Regel patientennah, damit sowohl das ein- als auch das ausgeatmete Gas analysiert werden kann.

Zur Messung des CO2-Gehalts in der Atemluft befindet sich im Beatmungsgerät typischerweise eine Messküvette, in der das Probengas mit nahinfrarotem Licht durchleuchtet wird. Das CO2 in dem Probengas absorbiert einen Teil des Lichtes, sodass anhand der verbleibenden Lichtstärke der CO2-Gehalt in der Atemluft bestimmbar ist. Diese Sensortechnologie ist vergleichsweise schnell, sodass die CO2-Konzentration in der Atemluft über den gesamte Atemzyklus exakt bestimmbar ist (die Anstiegszeit t(10%-90%) derartiger CO2-Sensoren beträgt etwa 90ms).

Die US 2013/165806 A1 offenbart die Detektion einer Atemphase und die atemphasenspezifische Auswertung des CO2-Gehalts einer Gasprobe der Atemluft eines Patienten.

Die US 2006/201507 A1 offenbart ein System zur Messung des Sauerstoffverbrauchs, wobei die Atemphase detektiert und eine Gasprobe gemessen wird.

Die DE 10 2008 022761 A1 offenbart eine Vorrichtung zur Gewinnung und Untersuchung von Atemgasproben, wobei die Atemphasenermittlung mithilfe eines CO2-Sensors erfolgt und eine atemphasenspezifische Untersuchung der Gasproben vorgenommen wird.

Zur Messung des O2-Gehalts in der Atemluft gibt es grundsätzlich verschiedene Sensortechnologien, wie beispielsweise paramagnetische Sensoren, galvanische Zellen, Zirkoniumdioxidsensoren, Fluoreszenzsensoren und die Laserspektroskopie. Die verschiedenen Sensoren unterscheiden sich weiterhin in ihrer Größe, Ansprechzeit, Messgenauigkeit, Verfügbarkeit, Lebenszeit und dem Preis, wobei schnellere Sensoren meist teurer sind als langsamere Sensoren.

Relativ kleine und günstige Sensoren zur Messung des O2-Gehaltes sind beispielsweise galvanische Sauerstoffsensoren, bei denen das Gas über eine dünne Membran an die Kathode gelangt, an der eine erste Reaktion stattfindet. Über das flüssige Elektrolyt des Sensors gelangen die entstandenen Ladungsträger zur Kathode, wo eine weitere Reaktion stattfindet. Dabei wird die Beschichtung der Anode mit jeder Reaktion abgenutzt, weshalb auch von einem verbrauchenden Sensor gesprochen wird. Der entstandene Strom ist proportional zur O2-Konzentration im Probengas, aber auch abhängig vom Druck und der Temperatur. Derartige galvanische O2-Sensoren benötigen keine externe Energiequelle, sind jedoch - je nach Gebrauch und Ansprechzeit des Sensors - nur ein bis zwei Jahre verwendbar. Die Anstiegszeit t(10%-90%) solcher Sensoren beträgt typischerweise etwa 2s, sodass eine Nutzung für eine zeitlich hochaufgelöste Messung des O2-Gehalts nicht möglich ist.

Zur Messung des O2-Gehalts in der Atemluft ist die Verwendung schneller O2-Sensoren insbesondere in der Notfallbeatmung aus Platz- und/oder Kostengründen nicht üblich. Trotzdem ist es für das medizinische Personal von großem Interesse, die endtidale Sauerstoffkonzentration (etO2) zu bestimmen, um Rückschlüsse über die Menge des bei der Beatmung eines Patienten von diesem aufgenommenen Sauerstoffs bzw. des nicht von diesem aufgenommenen und somit ausgeatmeten Sauerstoffs ziehen zu können.

Insbesondere bei der Präoxygenierung, einem Vorgang, bei dem Stickstoff aus der Lunge eines Patienten gewaschen und mit Sauerstoff ersetzt wird, spielt der etO2-Wert eine wichtige Rolle. Mithilfe des etO2-Wertes kann dabei der Endzeitpunkt des Vorgangs anhand gemessener Daten bestimmt werden, sodass dieser nicht aus Erfahrungswerten abgeleitet werden muss, was ggf. ungenau für den individuellen Patienten sein kann.

Auch die Bestimmung des Gehalts anderer Gase in der Atemluft, wie beispielsweise VOCs (flüchtige organische Stoffe), Wasserstoff oder Anästhesiegasen, ist in bestimmten Anwendungen von Interesse.

Eine Aufgabe der Erfindung ist es daher, eine verbesserte Vorrichtung zur Messung des Gehalts zumindest eines Gases in Ausatemluft zu schaffen.

Diese Aufgabe wird erfindungsgemäß durch eine Vorrichtung zur Messung des Gehalts zumindest eines Gases in Ausatemluft gemäß Patentanspruch 1 gelöst.

Eine weitere Aufgabe der Erfindung ist es, eine Vorrichtung zur Messung des Gehalts zumindest eines Gases in Ausatemluft zu schaffen, die die Bestimmung des Gehalts dieses Gases auch mit einem Sensor mit einer langsamen Ansprechzeit in einer für die jeweilige Anwendung hinreichenden Art und Weise ermöglicht.

Diese Aufgabe wird erfindungsgemäß durch eine Vorrichtung zur Messung des Gehalts zumindest eines Gases in Ausatemluft gemäß Patentanspruch 1 gelöst.

Eine weitere Aufgabe der Erfindung ist es, eine verbesserte Vorrichtung zur Beatmung zu schaffen.

Diese Aufgabe wird erfindungsgemäß durch eine Vorrichtung zur Beatmung gemäß Patentanspruch 6 gelöst.

Eine weitere Aufgabe der Erfindung ist es, ein Verfahren zur Messung des Gehalts zumindest eines Gases in Ausatemluft zu schaffen, das die Bestimmung des Gehalts dieses Gases auch mit einem Sensor mit einer langsamen Ansprechzeit in einer für die jeweilige Anwendung hinreichenden Art und Weise ermöglicht.

Diese Aufgabe wird erfindungsgemäß durch ein Verfahren zur Messung des Gehalts zumindest eines Gases in Ausatemluft gemäß Patentanspruch 7 gelöst.

Die nachfolgend offenbarten Merkmale einer Vorrichtung zur Messung des Gehalts zumindest eines Gases in Ausatemluft sind sowohl einzeln als auch in allen ausführbaren Kombinationen Teil der Erfindung.

Eine erfindungsgemäße Vorrichtung zur Messung des Gehalts zumindest eines Gases in Ausatemluft weist zumindest eine Vorrichtung zur Bestimmung der Atemphase und zusätzlich zumindest einen Gassensor, sowie mindestens eine Steuereinheit und mindestens ein steuerbares Ventil und eine Pumpvorrichtung auf.

Die Vorrichtung zur Bestimmung der Atemphase ist dabei zur Bestimmung der aktuellen Atemphase und zur Erkennung eines definierten Bereichs im Atemzyklus eines Patienten ausgebildet. Dazu weist die Vorrichtung zur Bestimmung der Atemphase mindestens einen geeigneten Atemphasensensor auf.

In einer Ausführungsform der Erfindung weist die Vorrichtung zur Bestimmung der Atemphase mindestens einen als ein CO2-Sensor, ein O2-Sensor, ein Feuchte-Sensor, ein Temperatursensor, ein Drucksensor oder ein Flusssensor ausgebildeten Atemphasensensor auf.

Der mindestens eine Atemphasensensor der Vorrichtung zur Bestimmung der Atemphase ist dabei zur Messung nach dem Nebenstrom- oder dem Hauptstromverfahren bezogen auf dem Hauptstrom der Atemluft angeordnet.

Bei der Verwendung eines Druck- oder eines Flusssensors in der Vorrichtung zur Bestimmung der Atemphase ist der jeweilige Atemphasensensor in einer bevorzugten Ausführungsform der Erfindung zur Messung im Hauptstromverfahren angeordnet. Auch die Verwendung von CO2-, Temperatur- oder Feuchtesensoren ist zur Messung im Hauptstrom möglich.

Der Volumenstrom der Atemluft zum Patienten hin liegt in einer Größenordnung von etwa 0 bis 100 l/min. Die Absaugung von Probengas für eine Messung im Nebenstromverfahren beträgt in Ausführungsformen der Erfindung etwa 100 ml/min.

Bei der Verwendung eines CO2-, eines O2-, eines Feuchte- oder eines Temperatursensors als ein Atemphasensensor in der Vorrichtung zur Bestimmung der Atemphase ist der jeweilige Atemphasensensor in einer bevorzugten Ausführungsform der Erfindung zur Messung im Nebenstromverfahren angeordnet.

In einer vorteilhaften Ausführungsform der Erfindung weist die Vorrichtung zur Messung des Gehalts zumindest eines Gases in Ausatemluft mindestens einen Wasserfilter oder eine sonstige Einrichtung zur Trocknung des Probengases auf, sodass eine Kondensation des in der Regel insbesondere in der Ausatemluft enthaltenen Wasserdampfs im Bereich der Messeinrichtung und eine damit einhergehende Beeinträchtigung der Messungen vermieden wird. Insbesondere ist die Verwendung mindestens eines Wasserfilters oder einer Einrichtung zur Trocknung des Probengases bei der Messung im Nebenstrom vorteilhaft. Eine Feuchtemessung nach der Trocknung des Probengases ist für die Bestimmung der Atemphase selbstverständlich wenig hilfreich.

Die Konzentration von CO2 und O2 ändert sich, wie bereits vorstehend beschrieben, im Verlauf des Atemzyklus. Auch die Feuchte, Temperatur sowie der Druck und der Fluss der Atemluft sind abhängig vom Atemzyklus. So ist die ausgeatmete Luft in der Regel feuchter und wärmer als die dem Patienten zugeführte Luft, zudem strömt diese in die entgegengesetzte Richtung.

Mithilfe einer Vorrichtung zur Bestimmung der Atemphase gemäß der vorliegenden Erfindung ist mindestens einer der vorstehend genannten Messwerte mithilfe eines entsprechenden, zeitlich hochauflösenden Atemphasensensors (schneller Sensor), erfassbar und der Messwertverlauf dahingehend auswertbar, in welcher Atemphase des Atemzyklus sich die Atmung bzw. die Beatmung eines Patienten aktuell befindet.

Neben der Inspiratons- und der Exspirationsphase als solche ist dabei insbesondere die Erkennung des endtidalen Bereichs am Ende der Exspirationsphase von Interesse.

In einer besonders vorteilhaften Ausführungsform der Erfindung ist die Vorrichtung zur Bestimmung der Atemphase daher zur Erkennung des endtidalen Bereichs ausgebildet.

Mithilfe der Steuereinheit ist in Abhängigkeit der detektierten Atemphase das mindestens eine steuerbare Ventil und/oder die Pumpvorrichtung ansteuerbar.

In einer vorteilhaften Ausführungsform der Erfindung weist das mindestens eine steuerbare Ventil zumindest einen öffenbaren und verschließbaren Ventilweg auf, über den das zu vermessende Gas zu mindestens einem Gassensor zuleitbar ist.

Zusätzlich ist die Pumpvorrichtung mithilfe der Steuereinheit derart ansteuerbar, dass das zu vermessende Gas zu mindestens einem Gassensor zuleitbar ist.

In einer besonders bevorzugten Ausführungsform der Erfindung ist die Vorrichtung zur Bestimmung der Atemphase zur Bestimmung des endtidalen Bereichs ausgebildet und das steuerbare Ventil mithilfe der Steuereinheit so ansteuerbar, dass der mindestens eine Ventilweg des steuerbaren Ventils für die Dauer des endtidalen Bereichs der Exspirationsphase geöffnet ist und mit dem Beginn der Inspirationsphase verschlossen ist, sodass die in den Bereich des mindestens einen Gassensors zugeleitete Gasprobe der Atemluft aus dem endtidalen Bereich der Exspirationsphase entstammt.

Sinngemäß ist die vorstehend beschriebene Logik zum Öffnen oder Schließen eines oder mehrerer Ventilwege des steuerbaren Ventils in Abhängigkeit beliebiger Bereiche (Atemphasen) im Atemzyklus erfindungsgemäß möglich.

In bevorzugten Ausführungsformen der Erfindung ist die Vorrichtung zur Bestimmung der Atemphase zur Bestimmung des endtidalen Bereichs ausgebildet und die Pumpvorrichtung mithilfe der Steuereinheit so ansteuerbar, dass die Pumpvorrichtung für die Dauer des endtidalen Bereichs der Exspirationsphase Probengas in den Bereich des Gassensors fördert und mit dem Beginn der Inspirationsphase abschaltet, sodass die in den Bereich des mindestens einen Gassensors zugeleitete Gasprobe der Atemluft aus dem endtidalen Bereich der Exspirationsphase entstammt.

Der vorstehend beschriebene Aufbau einer erfindungsgemäßen Vorrichtung zur Messung des Gehalts zumindest eines Gases in Ausatemluft ermöglicht so das gezielte Zuführen einer Probe der Atemluft aus einer definierten Atemphase und anschließend einen längeren Zeitraum, in dem die Atemluftprobe mithilfe des mindestens einen Gassensors zur Bestimmung des Gehalts zumindest eines Gases in der Atemluft vermessbar ist. Dadurch ist die Verwendung von Gassensoren mit einer längeren Anstiegszeit und/oder einer höheren Genauigkeit möglich (langsamer Sensor).

Gemäss der Erfindung ist mindestens ein Gassensor als ein O2-Sensor ausgebildet, sodass der endtidale O2-Gehalt der Atemluft (etO2-Wert) bestimmbar ist.

Die Frequenz des Atemzyklus liegt bei Erwachsenen typisch bei 10/min, bei Kindern bei 40/min und kann im Rahmen einer Herz-Lungen-Wiederbelebung bei bis zu 110/min liegen. Entsprechend liegt die Periode des Atemzyklus etwa zwischen 540ms (Herz-Lungen-Wiederbelebung) und 6s (typ. Atemzyklus eines Erwachsenen).

Die Menge der zur zuverlässigen Bestimmung der Atemphase erforderlichen Messwerte ist von der Länge des interessierenden Bereichs abhängig. Je nach Frequenz bzw. Periode des gewünschten Beatmungsmodus (z.B. Erwachsener, Kind, Herz-Lungen-Wiederbelebung) ist somit die maximal für einen Gassensor zulässige Anstiegszeit bestimmbar.

Die maximal für einen schnellen Sensor der Atemphasenerkennung zulässige Anstiegszeit ist in einer Ausführungsform der Erfindung bestimmbar als t₁₀₋₉₀ = 60 / (10 · fₘₐₓ), wobei von 10 Messwerten je Atemzug ausgegangen wird und fₘₐₓ die maximale Frequenz der zu erwartenden Atmung bzw. Beatmung in 1/min angibt. Für die Beatmung eines "Riesen" mit einer erwarten Frequenz fₘₐₓ von 5/min ergibt sich somit eine maximale Anstiegszeit t₁₀₋₉₀ von 1200ms, für einen Erwachsenen mit einer erwarteten Frequenz fₘₐₓ von 10/min eine maximale Anstiegszeit t₁₀₋₉₀ von 600ms, für ein Kind mit einer erwarteten Frequenz fₘₐₓ von 20/min eine maximale Anstiegszeit t₁₀₋₉₀ von 300ms, für einen Säugling mit einer erwarteten Frequenz fₘₐₓ von 50/min eine maximale Anstiegszeit t₁₀₋₉₀ von 120ms, für eine Reanimation mit einer Herzdruckmassage mit einer erwarteten Frequenz fₘₐₓ von 100/min eine maximale Anstiegszeit t₁₀₋₉₀ von 60ms und bei einer etwas zu schnellen Herzdruckmassage mit einer erwarteten Frequenz fₘₐₓ von 110/min eine maximale Anstiegszeit t₁₀₋₉₀ von 54,5ms.

Ein zeitlich hochauflösender Sensor bzw. schneller Sensor im Sinne dieser Anmeldung ist somit bezogen auf die Atmung oder Beatmung eines Erwachsenen ein Sensor mit einer Anstiegszeit in einem Bereich kleiner als etwa 500ms und ein Sensor mit einer längeren Anstiegszeit bzw. langsamer Sensor im Sinne dieser Anmeldung weist eine Anstiegszeit von mehr als etwa 500ms auf.

In einer vorteilhaften Ausführungsform der Erfindung wird zur Atemphasenerkennung ein schneller Sensor mit einer Anstiegszeit von maximal 90ms verwendet.

In einer besonders vorteilhaften Ausführungsform der Erfindung wird zur Atemphasenerkennung ein schneller Sensor mit einer Anstiegszeit von maximal 60ms verwendet.

Ist die erfindungsgemäße Vorrichtung zur Messung des Gehalts zumindest eines Gases in Ausatemluft zur Messung zumindest eines der mithilfe der Vorrichtung zur Bestimmung der Atemphase oder mithilfe des weiteren Gassensors erfassbaren Messwerte gemäß dem Nebenstromverfahren ausgebildet, so weist diese bevorzugt eine Pumpvorrichtung auf, mit der eine Gasprobe aus dem Hauptstrom heraus abpumpbar und in den Bereich des jeweiligen Sensors transportierbar ist.

In einer Ausführungsform der Erfindung ist die Pumpvorrichtung als eine Absaugpumpe ausgebildet.

In einer besonders bevorzugten Ausführungsform der Erfindung ist die Vorrichtung zur Messung des Gehalts zumindest eines Gases in Ausatemluft zusätzlich zur Messung des Gehalts zumindest eines Gases in der Einatemluft ausgebildet.

Dazu weist die Vorrichtung zur Messung des Gehalts zumindest eines Gases in Ausatemluft ein weiteres steuerbares Ventil auf, oder das steuerbare Ventil ist als ein Mehrwegeventil ausgebildet, wobei die Ventilwege mithilfe der Vorrichtung zur Bestimmung der Atemphase und der Steuereinheit korrespondierend zu definierten Atemphasen öffenbar oder verschließbar sind.

In einer Ausführungsform der Erfindung ist die Vorrichtung zur Messung des Gehalts zumindest eines Gases in Ausatemluft dabei zur inspiratorischen Sauerstoffmessung ausgebildet.

In einer besonders bevorzugten Ausführungsform der Erfindung wird zur Bestimmung des inspiratorischen Sauerstoffgehalts derselbe Sensor verwendet, der auch zur Bestimmung des endtidalen Sauerstoffgehalts eingesetzt wird.

Da die Zusammensetzung der zur Beatmung verwendeten Luft in der Regel nur geringen Schwankungen unterliegt, ist der Sauerstoffgehalt (oder der Gehalt eines anderen interessierenden Gases) der einem Patienten zugeführten Luft in der Zeit des Atemzyklus dem O2-Sensor (oder einem anderen, den Gehalt des interessierenden Gases messenden Gassensor) zuführbar, die nicht zur Bestimmung des etO2-Wertes (oder des Gehalts des anderen interessierenden Gases in der interessierenden Atemphase) benötigt wird, sodass mit dem Gassensor alternierend der Gehalt des jeweiligen Gases in der dem Patienten zugeführten Atemluft und in der Ausatemluft bestimmbar ist.

Eine erfindungsgemäße Vorrichtung zur Beatmung weist mindestens eine erfindungsgemäße Vorrichtung zur Messung des Gehalts zumindest eines Gases in Ausatemluft gemäß der vorstehenden Beschreibung auf.

Die nachfolgend offenbarten Merkmale eines Verfahrens zur Messung des Gehalts zumindest eines Gases in Ausatemluft sind sowohl einzeln als auch in allen ausführbaren Kombinationen Teil der Erfindung.

Ein erfindungsgemäßes Verfahren zur Messung des Gehalts zumindest eines Gases in Ausatemluft weist zumindest die folgenden Verfahrensschritte auf:
1. Bestimmung der Atemphase
2. Erkennung, ob eine vorbestimmte Atemphase in einem Atemzyklus vorliegt
3. Zuführen einer Gasprobe zu mindestens einem Gassensor
4. Isolation der Gasprobe im Bereich des mindestens einen Gassensors
5. Messung der zugeführten Gasprobe mithilfe des mindestens einen Gassensors
6. Aufheben der Isolation der Gasprobe

In vorteilhaften Ausführungsformen laufen die vorstehend genannten Verfahrensschritte in einer Schleife bis zur Beendigung der Messung sequentiell kontinuierlich ab.

In einer besonders bevorzugten Ausführungsform des erfindungsgemäßen Verfahrens erfolgt die Bestimmung der Atemphase kontinuierlich und parallel zu den übrigen Verfahrensschritten.

Das Zuführen der Gasprobe zum zumindest einen Gassensor erfolgt in erfindungsgemäßen Ausführungsformen des Verfahrens bis zur Isolation der Gasprobe ggf. kontinuierlich und unabhängig von der Atemphase, d.h. dieser Schritt kann auch vor der Erkennung der mindestens einen vorbestimmten Atemphase und ggf. auch vor der (erstmaligen) Bestimmung der Atemphase erfolgen.

Die Bestimmung der Atemphase erfolgt in einer bevorzugten Ausführungsform der Erfindung durch die kontinuierlich Auswertung von zeitlich abfolgend erfassten Messwerten zumindest eines Sensors einer Vorrichtung zur Bestimmung der Atemphase, wobei zumindest ein Sensor zur zeitlich hochauflösenden Messung des CO2-Gehalts, des O2-Gehalts, der Feuchte, der Temperatur, des Drucks und/oder des Flusses des Atemgases, insbesondere der Ausatemluft, verwendet wird.

Die Bestimmung der Atemphase erfolgt in Ausführungsformen des Verfahrens durch Messungen im Hauptstromprinzip.

In anderen Ausführungsformen des erfindungsgemäßen Verfahrens erfolgt die Bestimmung der Atemphase durch Messungen im Nebenstromprinzip.

Dabei wird die jeweilige Atemphase in Ausführungsformen des Verfahrens durch das Überschreiten oder das Unterschreiten eines vorgegebenen Schwellwerts bzw. mehrerer vorgegebener Schwellwerte durch die Messwerte erkannt.

Für verschiedene Messwerte sind nachfolgend beispielhaft Schwellwerte zur Erkennung der Atemphase angegeben.

Wird ein CO2-Sensor zur Bestimmung der Atemphase verwendet, so zeigt in einer Ausführungsform der Erfindung ein Wert für CO2 > 2,5 Vol.-% die Exspirationsphase an. Dieser Wert liegt während der Exspiration typ. bei 5 Vol.-%. Wenn der Wert anschließend wieder unter 0,5 Vol.-% CO2 fällt, wird eine Inspirationsphase erkannt.

In einer anderen Ausführungsform der Erfindung ist der Schwellwert des CO2-Gehalts in der Atemluft zur Bestimmung der Inspirationsphase auf etwa 0,8 CO2max und damit auf etwa 3,6 Vol.-% CO2 gesetzt. Dies ermöglicht eine schnellere Detektion der Inspirationsphase als eine Schwelle bei 0,5 Vol.-% CO2, birgt jedoch in Abhängigkeit der bei der Beatmung des Patienten tatsächlich erreichten CO2-Gehalte der Atemluft ggf. Probleme mit der Robustheit der Atemphasenerkennung.

Wird ein O2-Sensor zur Atemphasenerkennung verwendet, so zeigt bei der Beatmung eines Patienten mit Raumluft in einer Ausführungsform der Erfindung ein Wert für O2 > 20 Vol.-% eine Inspirationsphase und ein Wert für O2 < 19 Vol.-% eine Exspirationsphase an.

Wird ein O2-Sensor zur Atemphasenerkennung verwendet, so zeigt bei der Beatmung eines Patienten mit reinem Sauerstoff in einer Ausführungsform der Erfindung ein Wert für O2 > 98 % eine Inspirationsphase und ein Wert für O2 < 98 Vol.-% eine Exspirationsphase an.

Wird für die Atemphasenerkennung ein Flusssensor im Hauptstrom verwendet, so zeigt in einer Ausführungsform der Erfindung ein Wert für den Fluss > 0,5 l/min (in Patientenrichtung) eine Inspirationsphase und ein Wert für den Fluss < -0,5 l/min (vom Patienten kommend) eine Exspirationsphase an.

Wird für die Atemphasenerkennung ein Gas-Temperatursensor im Hauptstrom verwendet, so zeigt in einer Ausführungsform der Erfindung ein Wert für die Temperatur T > 32°C eine Exspirationsphase und ein Wert für die Temperatur T < 28°C eine Inspirationsphase an.

Wird für die Atemphasenerkennung ein Feuchte-Sensor zur Messung der relativen Feuchtigkeit (rH) verwendet so zeigt in einer Ausführungsform der Erfindung ein Wert für rH > 90% eine Exspirationsphase an, weil feuchtes Gas ausgeatmet wird, und ein Wert für rH < 85% eine Inspirationsphase an.

In anderen Ausführungsformen der Erfindung erfolgt die Erkennung der Atemphase durch einen Abgleich mit zuvor erfassten Messwerten zumindest eines Atemzyklus, bevorzugt mehrerer Atemzyklen.

In Ausführungsformen der Erfindung erfolgt die Erkennung der Atemphase mithilfe dynamisch anpassbarer Schwellwerte, wobei die Anpassung der Schwellwerte basierend auf zumindest einem erfassten Parameter erfolgt.

Auch die Verwendung von Algorithmen, wie beispielsweise einer Fuzzylogik, ist in erfindungsgemäßen Ausführungsformen zur Erkennung der Atemphase denkbar. Andere Ausführungsformen nutzen zur Erkennung der Atemphase die erste oder die zweite Ableitung der jeweils betrachteten Messwertsignale.

Jedenfalls erfolgt die Isolation der Gasprobe im Bereich des mindestens einen Gassensors in Abhängigkeit der Atemphase nur, wenn eine definierte Atemphase (z.B. der endtidale Bereich) erkannt wurde.

Die Erkennung des endtidalen Bereichs erfolgt in vorteilhaften Ausführungsformen durch die Anwendung einer Verzögerung nach der Detektion des Endes der Exspirationsphase bzw. des Beginns der Inspirationsphase. Der endtidale Bereich ist der Bereich etwa 200ms bis 50ms vor dem Ende der Exspirationsphase, sodass durch ein entsprechendes Ansteuern des steuerbaren Ventils und/oder der Pumpvorrichtung das Atemgas aus dem endtidalen Bereich in der Exspirationsphase im Bereich des Gassensors isoliert wird.

In einer bevorzugten Ausführungsform der Erfindung wird das Probengas aus den letzten 30 % der Exspirationsphase zur Isolation in den Bereich des Gassensors geleitet.

Die Aufhebung der Isolation der Gasprobe im Bereich des mindestens einen Gassensors erfolgt, sobald die Messung des Gehalts des interessierenden Gases in der Gasprobe abgeschlossen ist und/oder sobald eine vorgegebene Zeit abgelaufen ist und/oder sobald ein externes Signal dies vorgibt.

In einer Ausführungsform des erfindungsgemäßen Verfahrens erfolgt die Isolation der Gasprobe im Bereich des mindestens einen Gassensors durch das Schließen zumindest eines Ventilwegs eines steuerbaren Ventils und/oder durch das Abschalten der Pumpvorrichtung. Das Aufheben der Isolation der Gasprobe im Bereich des mindestens einen Gassensors erfolgt in dieser Ausführungsform durch das Öffnen des zumindest eines Ventilwegs des steuerbaren Ventils und/oder das Einschalten der Pumpvorrichtung.

Die Zufuhr des zu analysierenden Atemgases in den Bereich des Gassensors erfolgt in Ausführungsformen der Erfindung mit Ausnahme der Isolationsphase kontinuierlich oder gezielt unmittelbar vor der Isolationsphase, sodass gewährleistet ist, dass während der Isolationsphase im Bereich des mindestens einen Gassensors Atemgas aus der interessierenden Atemphase vorhanden ist.

Ein in einem Leitungssystem ggf. vorhandenes Totvolumen ist bei der gezielten Zuführung des Atemgases unmittelbar vor der Isolationsphase zu beachten und wird in solchen Ausführungsformen des Verfahrens entsprechend vor der Isolationsphase aus dem Bereich des mindestens einen Gassensors abgeleitet. Dies wird beispielsweise durch das Abwarten einer vordefinierten Zeit (Verzögerung) nach der Erkennung der entsprechenden Atemphase realisiert, die in Abhängigkeit der Flussgeschwindigkeit des Probengases bzw. der Pumpleistung der Pumpvorrichtung und dem Leitungsvolumen der das Probengas führenden Leitung und der Geometrie der Messvorrichtung zu wählen ist.

In Ausführungsformen des erfindungsgemäßen Verfahrens erfolgt die Detektion der Atemphase und die Isolation der Gasprobe nicht in jedem Atemzyklus, sondern regelmäßig beispielweise in jedem x. Atemzyklus (x ∈ N, x ≠1).

Dabei wird in bevorzugten Ausführungsformen des Verfahrens die jeweils isolierte Gasprobe vermessen und ein gleitender Mittelwert über die Messwerte der einzelnen erfassten Atemhübe gebildet.

In einer bevorzugten Ausführungsform ist das erfindungsgemäße Verfahren zur Bestimmung des endtidalen O2-Gehalts (etO2-Wert) in der Atemluft ausgebildet.

Mithilfe der erfindungsgemäßen Vorrichtung zur Messung des Gehalts zumindest eines Gases in Ausatemluft sowie des erfindungsgemäßen Verfahrens zur Messung des Gehalts zumindest eines Gases in Ausatemluft bzw. einer erfindungsgemäßen Vorrichtung zur Beatmung ist somit durch die Kombination eines schnellen Atemphasensensors mit einem in Abhängigkeit der detektierten Atemphase steuerbaren Ventil die Verwendung eines langsameren Gassensors zur Messung des Gehalts eines bestimmten Gases in der Atemluft während eines bestimmten Zeitabschnitts im Atemzyklus ermöglicht, sodass die Verwendung kostengünstigerer und/oder kleinerer und/oder genauerer Gassensoren ermöglicht wird.

In den nachfolgend beschriebenen Figuren sind beispielhafte Ausführungsformen der Erfindung dargestellt. Es zeigen:
- Figur 1:: Ein schematisches Blockschaltbild einer erfindungsgemäßen Vorrichtung zur Messung des Gehalts zumindest eines Gases in Ausatemluft,
- Figur 2:: Ein weiteres schematisches Blockschaltbild einer Ausführungsform einer erfindungsgemäßen Vorrichtung zur Messung des Gehalts zumindest eines Gases in Ausatemluft mit weiteren Details,
- Figur 3:: Eine perspektivische Ansicht einer Explosionszeichnung eines Teils einer Vorrichtung zur Beatmung mit einer Einrichtung zur Entnahme einer Gasprobe aus dem Atemluftstrom,
- Figur 4:: Ein schematisches Ablaufdiagramm einer Ausführungsform eines erfindungsgemäßen Verfahrens zur Messung des Gehalts zumindest eines Gases in Ausatemluft und
- Figur 5:: Ein Diagramm zur Veranschaulichung des Verlaufs verschiedener Messgrößen über einen Atemzyklus.

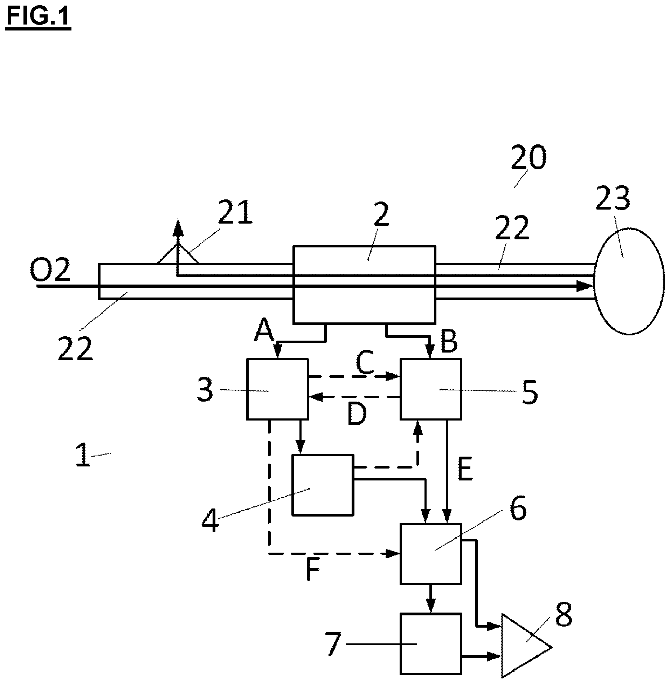

In Figur 1 ist ein schematisches Blockschaltbild einer erfindungsgemäßen Ausführungsform einer Vorrichtung zur Messung des Gehalts zumindest eines Gases in Ausatemluft (1) im Bereich einer ausschnittweise gezeigten Vorrichtung zur Beatmung (20) dargestellt.

Die Vorrichtung zur Messung des Gehalts zumindest eines Gases in Ausatemluft (1) ist über eine Anschlusseinrichtung (2) mit dem Atemluftstrom der Vorrichtung zur Beatmung (20) patientennah verbunden.

Die Anschlusseinrichtung (2) ist in der dargestellten Ausführungsform dabei als ein Teil der Atemluftleitung (22) ausgebildet und zwischen dem Patientenventil (21) und der Maske (23) der Vorrichtung zur Beatmung (20) angeordnet, sodass in der Inspirationsphase die zur Maske (23) bzw. zu einem Patienten hin geleitete Atemluft genauso wie die in der Exspirationsphase von der Maske (23) bzw. vom Patienten zum Patientenventil (21) hin geleitete Atemluft durch die Anschlusseinrichtung (2) hindurch strömt.

Die von der Vorrichtung zur Beatmung (20) durch die Atemluftleitung (22) zur Beatmung eines Patienten hindurch geleitete Atemluft ist in der Darstellung mit O2 gekennzeichnet und umfasst sowohl die Verwendung von Umgebungsluft zur Beatmung als auch die Verwendung von mit Sauerstoff angereicherter Luft.

Die Vorrichtung zur Messung des Gehalts zumindest eines Gases in Ausatemluft (1) weist weiterhin eine Vorrichtung zur Bestimmung der Atemphase (3) auf, die mit der Anschlusseinrichtung (2) verbunden ist. Dabei sind sowohl Anordnungen, die eine Messung im Hauptstromverfahren, als auch Anordnungen, die eine Messung im Nebenstromverfahren realisieren, erfindungsgemäße Ausführungsformen.

Weiterhin weist die gezeigte Ausführungsform einer Vorrichtung zur Messung des Gehalts zumindest eines Gases in Ausatemluft (1) eine Steuereinrichtung (4), eine Pumpvorrichtung (5), ein steuerbares Ventil (6), einen Gassensor (7) und einen Auslass (8) auf.

Mithilfe der Pumpvorrichtung (5) ist im Bereich der Anschlusseinrichtung (2) eine Gasprobe aus dem Atemluftstrom abpumpbar.

Mithilfe der Steuereinrichtung (4) ist in Abhängigkeit der mithilfe der Vorrichtung zur Bestimmung der Atemphase (3) bestimmten Atemphase zumindest das steuerbare Ventil (6) ansteuerbar, sodass die mithilfe der Pumpvorrichtung (5) geförderte Gasprobe durch einen geöffneten Ventilweg hindurch zum Gassensor (7) oder direkt zum Auslass (8) leitbar ist.

Die gestrichelten Pfeile zeigen optionale Konfigurationen einer erfindungsgemäßen Vorrichtung zur Messung des Gehalts zumindest eines Gases in Ausatemluft (1), die nachstehend erläutert werden.

In einer ersten erfindungsgemäßen Abwandlung der in Figur 1 gezeigten Ausführungsform einer Vorrichtung zur Messung des Gehalts zumindest eines Gases in Ausatemluft (1) ist die Pumpvorrichtung (5) so mit der Vorrichtung zur Bestimmung der Atemphase (3) und der Anschlusseinrichtung (2) verbunden, dass mithilfe der Pumpvorrichtung (5) geförderte Gasproben stets auch durch die Vorrichtung zur Bestimmung der Atemphase (3) hindurch strömen.

Dabei ist die Pumpvorrichtung (5) entweder in Strömungsrichtung der Gasproben vor (Flussrichtung entlang der Pfeile B, D, F) oder hinter (Flussrichtung entlang der Pfeile A, C, E) der Vorrichtung zur Bestimmung der Atemphase (3) angeordnet.

In einer erfindungsgemäßen Ausführungsform ist auch die Pumpvorrichtung (5) mithilfe der Steuereinheit (4) in Abhängigkeit der Atemphase ansteuerbar.

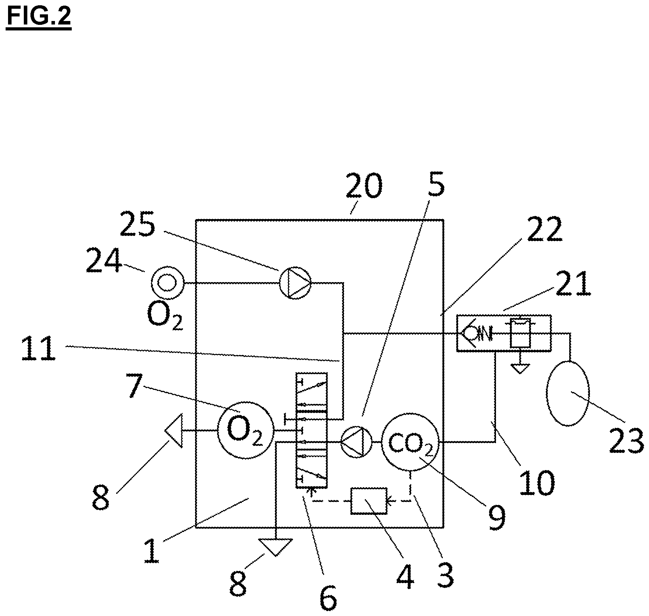

Figur 2 zeigt ein Blockschaltbild einer weiteren erfindungsgemäßen Ausführungsform einer Vorrichtung zur Messung des Gehalts zumindest eines Gases in Ausatemluft (1) bzw. einer erfindungsgemäßen Vorrichtung zur Beatmung (20).

Die Vorrichtung zur Messung des Gehalts zumindest eines Gases in Ausatemluft (1) ist zumindest teilweise in das Gehäuse der Vorrichtung zur Beatmung (20) integriert.

Im Bereich des Patientenventils (21) ist die Anschlussvorrichtung (2) angeordnet (nicht dargestellt) und über eine Entnahmeleitung (10) mit einem als ein CO2-Sensor ausgebildeten zeitlich hochauflösenden Atemphasensensor (9) der Vorrichtung zur Bestimmung der Atemphase (3) verbunden. Hinter dem Atemphasensensor (9) ist die Pumpvorrichtung (5) angeordnet, sodass Atemgasproben stets durch den Atemphasensensor (9) hindurchgeleitet werden. Hinter der Pumpvorrichtung (5) ist das steuerbare Ventil (6) angeordnet, das als ein Mehrwegeventil ausgebildet ist und in der gezeigten Ausführungsform zwei Eingänge und drei Ausgänge aufweist. Mit dem ersten Eingang ist die Pumpvorrichtung (5) verbunden, sodass mit dieser eine Gasprobe durch den ersten Eingang in das steuerbare Ventil (6) pumpbar ist. Mit dem zweiten Eingang ist ein Zweig (11) der Atemluftleitung (22) verbunden, der in Strömungsrichtung der Atemluft zum Patienten hin vor dem Patientenventil (21) angeordnet ist, sodass dieser stets nur die zur Beatmung verwendete Atemluft führt und nicht die Ausatemluft. Der erste Ausgang des steuerbaren Ventils (6) ist ein totes Ende (dicht verschlossen), an den zweiten Ausgang ist der Gassensor (7) angeschlossen und an den dritten Ausgang schließt sich der Auslass (8) an.

Mithilfe der Steuereinheit (4) ist das steuerbare Ventil (6) derart in Abhängigkeit der Atemphase schaltbar, dass nacheinander die Gasproben aus dem Bereich der Anschlussvorrichtung (2) und aus dem Zweig (11) der Atemluftleitung (22) zum Gassensor (7) oder direkt zum Auslass (8) leitbar sind.

Der Gassensor (7) ist in der dargestellten Ausführungsform der Erfindung als ein O2-Sensor ausgebildet, sodass je nach Ventilzustand des steuerbaren Ventils (6) der O2-Gehalt der zum Patienten hin geführten Atemluft oder der O2-Gehalt der Ausatemluft des Patienten messbar ist.

Die Vorrichtung zur Beatmung (20) weist weiterhin eine Atemluftquelle (24), die ggf. eine Sauerstoffquelle zur Anreicherung der Atemluft mit Sauerstoff aufweist, und ein Gebläse oder Ventil (25) auf, mit dem die Atemluft aktiv förderbar (Gebläse) oder der Fluss der Atemluft steuerbar (Ventil) ist.

Weiterhin weist die Vorrichtung zur Beatmung (20) eine Maske (23) auf, die zur Beatmung eines Patienten am Gesicht des Patienten anlegbar ist.

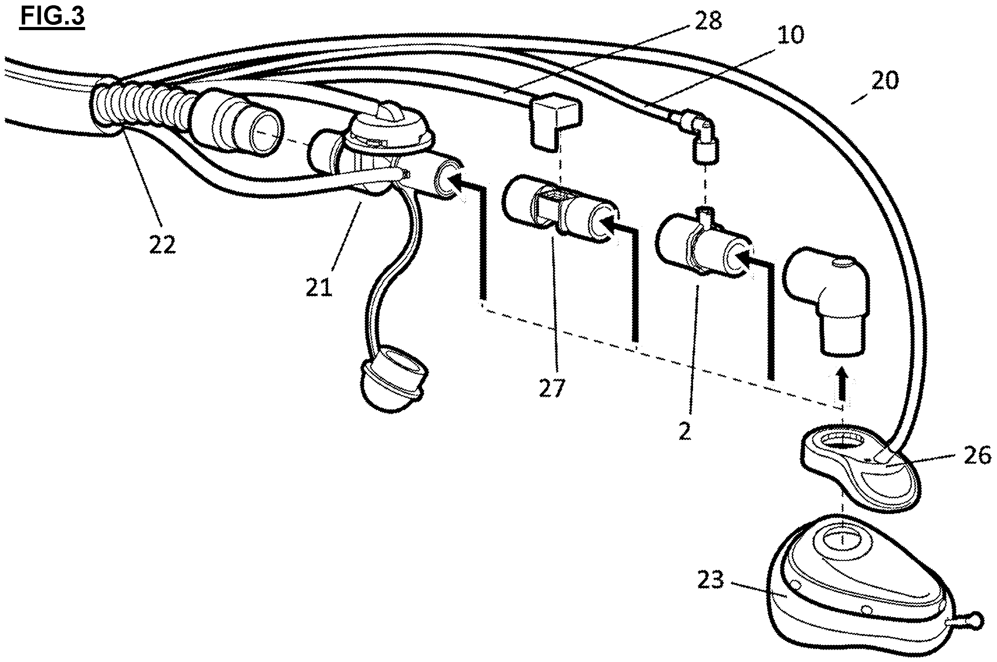

In Figur 3, die unter anderem der Veranschaulichung der Position der Anschlussvorrichtung (2) im System dient, ist in einer perspektivischen Ansicht eine Explosionszeichnung eines Teils einer erfindungsgemäßen Vorrichtung zur Beatmung (20) im Bereich des Patientenventils (21) dargestellt.

Das Patientenventil (21) ist mit dem Atemluftschlauch der Atemluftleitung (22) verbunden. Das Patientenventil (21) weist ein Rückschlagventil, eine Steuerleitung und einen Druckmessschlauch auf.

An das Patientenventil (21) schließt sich in Richtung zur Maske (23) hin ein Flusssensor (27) an, der über eine Anschlussleitung (28) mit einem Steuergerät bzw. einer Auswerteeinheit (nicht dargestellt) der Vorrichtung zur Beatmung (28) verbunden ist.

An den Flusssensor (27) schließt sich die Anschlusseinrichtung (2) an, die mit einer Entnahmeleitung (10) verbunden ist. Durch die Entnahmeleitung (10) sind Atemgasproben aus dem Bereich der Anschlusseinrichtung (2) zur hier nicht dargestellten Vorrichtung zur Messung des Gehalts zumindest eines Gases in Ausatemluft (1) hin leitbar.

Die Anschlusseinrichtung (2) ist über ein 90°-Verbindungsstück mit der Maske (23) bzw. mit einer an der Maske (23) angeordneten Taste (26) verbunden, wobei die Taste (26) zur Auslösung von Atemhüben der Vorrichtung zur Beatmung (20) nutzbar ist. Alternativ zu einer Taste (26) können erfindungsgemäße Ausführungsformen auch eine Fernbedienung aufweisen, mit der die entsprechende Funktion steuerbar ist.

Die dargestellte Konfiguration des Patientenschlauchsystems ist als ein Ein-Schlauchsystem ausgebildet. Davon abweichend ist die Erfindung auch in Zwei-Schlauchsystemen nutzbar, bei denen das Atemgas zunächst vom Patienten zum Beatmungsgerät geführt wird und dort über ein Ausatemventil abgelassen wird.

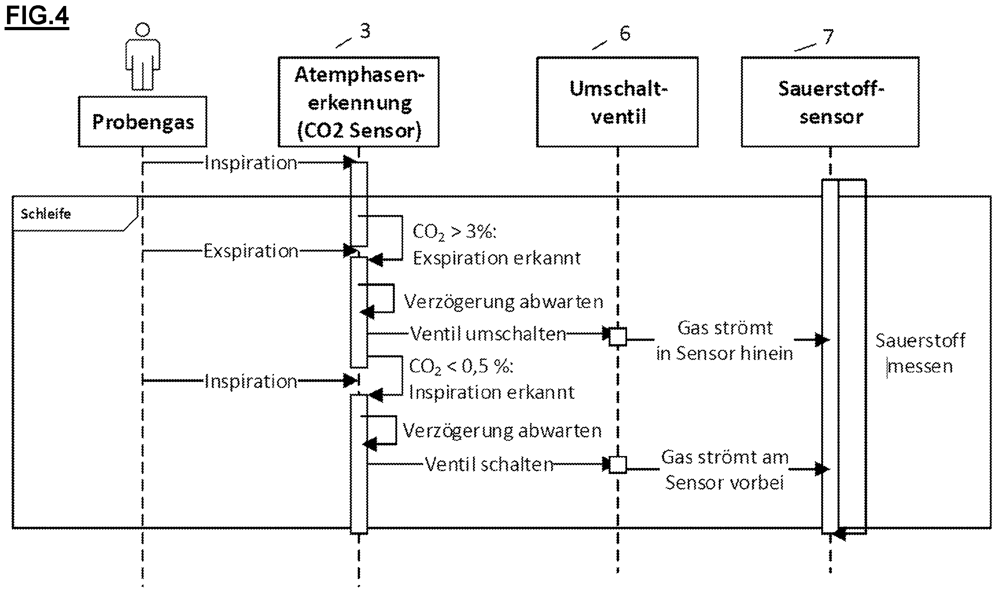

Figur 4 zeigt den Ablauf einer erfindungsgemäßen Ausführungsform eines Verfahrens zur Messung des Gehalts zumindest eines Gases in Ausatemluft.

Dabei wird anhand eines der Atemluft eines Patienten entnommenen Probengases mithilfe einer Vorrichtung zur Bestimmung der Atemphase (3) die Atemphase erkannt. Im vorliegenden Ausführungsbeispiels wird dabei der CO2-Gehalt des Probengases mithilfe eines als ein CO2-Sensor ausgebildeten Atemphasensensors (9) bestimmt und anhand von Schwellwerten bestimmt, welche Atemphase aktuell vorliegt. Der erste Schwellwert, der zur Detektion einer Exspirationsphase dient, liegt in dieser Ausführungsform des Verfahrens bei einem CO2-Gehalt von 3 Vol.-%, der überschritten werden muss.

Anschließend wird eine vordefinierte Zeitspanne (Verzögerung) abgewartet, bevor das steuerbare Ventil (6) umgeschaltet wird. Mit dem Umschalten des steuerbaren Ventils (6) strömt das Gas der Atemgasprobe in den Gassensor (7) hinein, der in dieser Ausführungsform als ein O2-Sensor ausgebildet ist.

Daraufhin wird mithilfe des Gassensors (7) der O2-Gehalt der Atemgasprobe gemessen.

Die Atemphasenerkennung läuft währenddessen kontinuierlich weiter, wobei die Detektion einer Inspirationsphase durch das Unterschreiten eines zweiten Schwellwerts erfolgt, der in dieser Ausführungsform des Verfahrens bei einem CO2-Gehalt von 0,5 Vol.-% liegt, der unterschritten werden muss.

Wird eine Inspirationsphase detektiert, wird eine definierte Zeit abgewartet (Verzögerung) und anschließend das steuerbare Ventil (6) umgeschaltet, sodass das Gas der Atemgasprobe nicht durch den Gassensor (7) hindurch strömt, bzw. am Gassensor (7) vorbei strömt.

Die zuvor erwähnte Isolationsphase ist dabei die Zeit, in der kein neues Probengas zum Gassensor (7) strömt, also die Zeit zwischen dem Umschalten des steuerbaren Ventils (6) nach der Detektion einer Inspirationsphase bis zum Umschalten des steuerbaren Ventils (6) nach der Detektion der darauffolgenden Exspirationsphase.

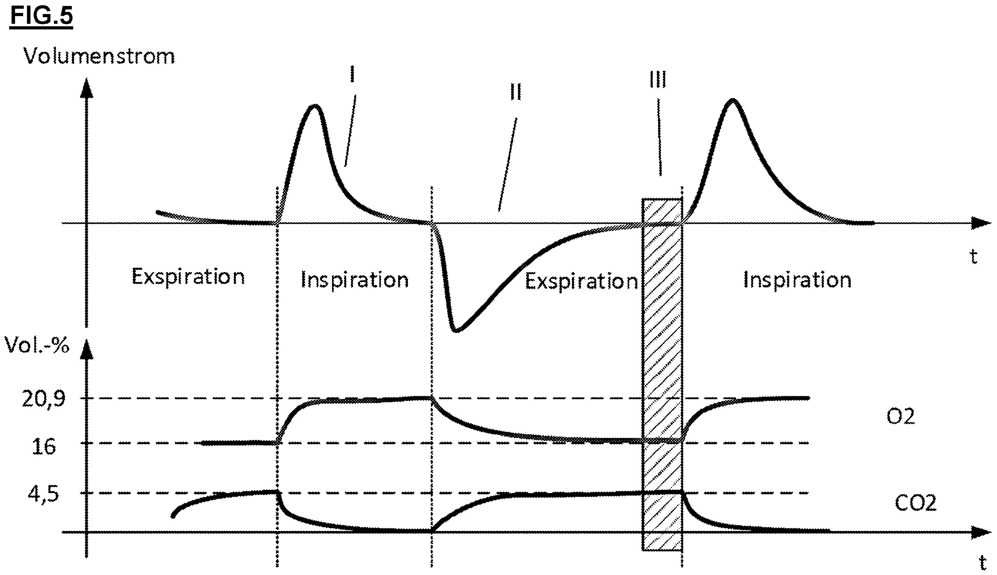

In Figur 5 ist in zwei verbundenen Diagrammen der Verlauf des Flusses der Atemluft und der CO2- sowie der O2-Gehalt der Atemluft über einen Atemzyklus hinweg dargestellt. Die Abszisse (horizontale Achse) ist dabei die Zeitachse und die Ordinate (vertikale Achse) der Volumenstrom [I/s] bzw. der Gasgehalt [Vol.-%].

Ein Atemzyklus ist dabei in die Phasen Inspirationsphase (I) und Exspirationsphase (II) unterteilt, wobei der letzte Abschnitt der Exspirationsphase (II) durch den endtidalen Bereich (III) gegeben ist.

Während der Inspirationsphase steigt der Volumenstrom des Atemgases zunächst steil an und sinkt anschließend langsam wieder. Der O2-Gehalt der Atemgasprobe steigt während der Verdrängung der Ausatemluft aus dem Gassensor vom Endwert der vorangegangenen Exspirationsphase (Il) von etwa 16 Vol.-% auf den O2-Gehalt der Atemluftquelle der Vorrichtung zur Beatmung, hier 20,9 Vol.-%, an. Parallel dazu sinkt der CO2-Gehalt der Atemgasprobe vom Endwert der vorangegangenen Exspirationsphase (Il) von etwa 4,5 Vol.-% auf nahezu 0 Vol.-%.

Während der Exspirationsphase (II) steigt der Volumenstrom in entgegengesetzter Richtung zunächst steil an und sinkt anschließen langsam wieder. Der O2-Gehalt der Atemgasprobe sinkt während der Verdrängung der zur Beatmung zum Patienten hin geleiteten Atemluft aus dem Gassensor vom Endwert der vorangegangenen Inspirationsphase (I) von etwa 20,9 Vol.-% auf den O2-Gehalt der Ausatemluft des Patienten, hier 16 Vol.-%, ab. Parallel dazu steigt der CO2-Gehalt der Atemgasprobe vom Endwert der vorangegangenen Inspirationsphase (I) von nahezu 0 Vol.-% auf etwa 4,5 Vol.-%.

Im endtidalen Bereich (III) erfahren die Werte des Volumenstromes sowie des O2- und des CO2-Gehalts nur noch sehr geringe Änderungen, sodass nahezu von konstanten Werten dieser Messgrößen im endtidalen Bereich ausgegangen werden kann. In Bezug auf den O2- und den CO2-Gehalt wird dabei von den bereits zuvor erwähnten Konzentrationsplateaus gesprochen.

## Patentansprüche

1. Vorrichtung zur Messung des Gehalts zumindest eines Gases in Ausatemluft (1) aufweisend mindestens eine Vorrichtung zur Bestimmung der Atemphase (3), mindestens eine Steuereinheit (4), mindestens ein steuerbares Mehrwegeventil (6), aufweisend mindestens einen Eingang und mindestens zwei Ausgänge, und mindestens eine Pumpvorrichtung (5) sowie mindestens einen Gassensor (7), wobei mithilfe der Vorrichtung zur Bestimmung der Atemphase (3) die aktuell vorliegende Atemphase in einem Atemzyklus bestimmbar ist und mit der Steuereinheit (4) das steuerbare Ventil (6) und die Pumpvorrichtung (5) so in Abhängigkeit der detektierten Atemphase ansteuerbar sind, dass eine Atemgasprobe der je nach Atemphase zu einem Patienten hin- oder von einem Patienten wegströmenden Atemluft durch den Gassensor (7) hindurchleitbar oder im Bereich des Gassensors (7) isolierbar ist, sodass mithilfe des Gassensors (7) der Gehalt eines Gases in der Ausatemluft in einem vorbestimmten Abschnitt des Atemzyklus bestimmbar ist, wobei die Vorrichtung zur Bestimmung der Atemphase (3) mindestens einen Atemphasensensor (9) aufweist, der als ein schneller Sensor mit einer Anstiegszeit von weniger als 500 ms ausgebildet ist, wobei mindestens ein Gassensor (7) als ein O2-Sensor ausgebildet ist und dass der O2-Sensor als ein langsamer Sensor mit einer Anstiegszeit von mehr als 500 ms zur Bestimmung des O2-Gehalts in einer Atemgasprobe ausgebildet ist und dass das steuerbare Mehrwegeventil (6) in Strömungsrichtung der Atemgasprobe hinter der Pumpvorrichtung (5) und vor dem Gassensor (7) angeordnet ist, wobei ein Eingang des Mehrwegeventils (6) mit der Pumpvorrichtung (5), ein Ausgang des Mehrwegeventils (6) mit dem Gassensor (7) und ein weiterer Ausgang des Mehrwegeventils (6) mit einem Auslass (8) verbunden ist, sodass die Atemgasprobe je nach Ventilstellung des steuerbaren Mehrwegeventils (6) direkt in den Gassensor (7) hinein oder zum Auslass (8) leitbar ist.

2. Vorrichtung (1) nach Anspruch 1, **dadurch gekennzeichnet, dass** die Vorrichtung zur Bestimmung der Atemphase (3) zur Messung im Hauptstrom ausgebildet ist.

3. Vorrichtung (1) nach einem der Ansprüche 1 und 2, **dadurch gekennzeichnet, dass** die Vorrichtung zur Bestimmung der Atemphase (3) zur Messung im Nebenstrom ausgebildet ist.

4. Vorrichtung (1) nach einem der Ansprüche 1 bis 3, **dadurch gekennzeichnet**, zumindest ein Atemphasensensor (9) als ein CO2-Sensor, ein Temperatur-Sensor, ein Feuchte-Sensor, ein Flusssensor oder als ein Drucksensor ausgebildet ist.

5. Vorrichtung (1) nach einem der vorstehenden Ansprüche, **dadurch gekennzeichnet, dass** das steuerbare Ventil (6) als ein Mehrwegeventil aufweisend mindestens zwei Eingänge und mindestens zwei Ausgänge ausgebildet ist, sodass in Abhängigkeit der Atemphase Atemgasproben von verschiedenen Punkten des Systems durch den Gassensor (7) hindurchleitbar bzw. im Bereich des Gassensors (7) isolierbar sind.

6. Vorrichtung zur Beatmung (20) aufweisend zumindest eine Vorrichtung zur Messung des Gehalts zumindest eines Gases in Ausatemluft (1) nach einem der vorstehenden Ansprüche.

7. Verfahren zur Messung des Gehalts zumindest eines Gases in Ausatemluft unter Verwendung einer Vorrichtung zur Messung des Gehalts zumindest eines Gases in Ausatemluft (1) nach einem der Ansprüche 1 bis 5 aufweisend zumindest die folgenden Verfahrensschritte:
a. Bestimmung einer Atemphase mit einem Atemphasensensor mit einer Anstiegszeit von weniger als 500 ms
b. Erkennung, ob eine vorbestimmte Atemphase in einem Atemzyklus vorliegt
c. Zuführen einer Gasprobe zu mindestens einem als ein O2-Sensor ausgebildeten Gassensor (7) mit einer Anstiegszeit von mehr als 500 ms
d. Isolation der Gasprobe im Bereich des mindestens einen Gassensors (7)
e. Messung der zugeführten Gasprobe mithilfe des mindestens einen Gassensors (7)
f. Aufheben der Isolation der Gasprobe.

8. Verfahren nach Anspruch 7, **dadurch gekennzeichnet, dass** die Bestimmung der Atemphase und die Erkennung, ob eine vorbestimmte Atemphase vorliegt, kontinuierlich und parallel zu den übrigen Verfahrensschritten erfolgt.

9. Verfahren nach einem der Ansprüche 7 und 8, **dadurch gekennzeichnet, dass** die Erkennung der vorbestimmten Atemphase(n) anhand von vorgegebenen Schwellwerten für die zur Bestimmung der Atemphase erfassten Messwerte erfolgt, die jeweils über- oder unterschritten werden müssen.

10. Verfahren nach einem der Ansprüche 7 bis 9, **dadurch gekennzeichnet, dass** die Atemphase mithilfe einer mindestens einen Atemphasensensor (9) aufweisenden Vorrichtung zur Bestimmung der Atemphase (3) erfolgt, wobei mithilfe des mindestens einen Atemphasensensors (9) der CO2-Gehalt, die Feuchte, die Temperatur, der Fluss und/oder der Druck der zu einem Patienten hin- oder von einem Patienten wegströmenden Atemluft gemessen wird.

11. Verfahren nach einem der Ansprüche 7 bis 10, **dadurch gekennzeichnet, dass** mithilfe des Gassensors (7) der endtidale Sauerstoffgehalt in der Ausatemluft eines Patienten gemessen wird.

## Claims

1. Device for measuring the content of at least one gas in exhaled air (1) having at least one device for determining the respiratory phase (3), at least one control unit (4), at least one controllable multiport valve (6), having at least one inlet and at least two outlets, and at least one pumping device (5) and at least one gas sensor (7), wherein the device for determining the respiratory phase (3) can be used to determine the currently present respiratory phase in a respiratory cycle and the controllable valve (6) and the pumping device (5) can thus be actuated by the control unit (4) as a function of the detected respiratory phase, in that a respiratory gas sample of the respiratory air flowing towards or away from a patient depending on the respiratory phase can be passed through the gas sensor (7) or can be isolated in the region of the gas sensor (7), so that the content of a gas in the exhaled air can be determined in a predetermined section of the respiratory cycle with the aid of the gas sensor (7), wherein the device for determining the respiratory phase (3) has at least one respiratory phase sensor (9) which designed as a fast sensor with a rise time of less than 500 ms, wherein at least one gas sensor (7) is designed as an O2 sensor and the O2 sensor is designed as a slow sensor with a rise time of more than 500 ms for determining the O2 content in a respiratory gas sample and the controllable multi-port valve (6) is arranged downstream of the pumping device (5) and upstream of the gas sensor (7) in the direction of flow of the respiratory gas sample, wherein an inlet of the multi-port valve (6) is connected to the pumping device (5), an outlet of the multi-port valve (6) is connected to the gas sensor (7) and a further outlet of the multi-port valve (6) is connected to an outlet (8), so that the respiratory gas sample can be fed directly into the gas sensor (7) or to the outlet (8) depending on the valve position of the controllable multi-port valve (6).

2. Device (1) according to claim 1, **characterized in that** the device for determining the respiratory phase (3) is designed for measurement in the main-stream.

3. Device (1) according to one of claims 1 and 2, **characterized in that** the device for determining the respiratory phase (3) is designed for measurement in the secondary-stream.

4. Device (1) according to one of claims 1 to 3, **characterized in that** at least one respiratory phase sensor (9) is designed as a CO2 sensor, a temperature sensor, a humidity sensor, a flow sensor or as a pressure sensor.

5. Device (1) according to one of the preceding claims, **characterized in that** the controllable valve (6) is designed as a multiport valve having at least two inlets and at least two outlets, so that, depending on the respiratory phase, respiratory gas samples from different points of the system can be passed through the gas sensor (7) or can be isolated in the region of the gas sensor (7).

6. Device for ventilation (20) comprising at least one device for measuring the content of at least one gas in exhaled air (1) according to one of the preceding claims.

7. Method for measuring the content of at least one gas in exhaled air using a device for measuring the content of at least one gas in exhaled air (1) according to one of claims 1 to 5, comprising at least the following method steps:
a. Determination of a respiratory phase with a respiratory phase sensor with a rise time of I ess than 500 ms
b. Detection of whether a predetermined respiratory phase is present in a respiratory cycle
c. Supply of a gas sample to at least one gas sensor (7) designed as an O2 sensor with a rise time of more than 500 ms
d. Isolation of the gas sample in the area of at least one gas sensor (7)
e. Measuring the supplied gas sample using at least one gas sensor (7)
f. Terminating the isolation of the gas sample.

8. Method according to claim 7, **characterized in that** the determination of the respiratory phase and the detection of whether a predetermined respiratory phase is present is carried out continuously and in parallel with the other method steps.

9. Method according to one of claims 7 and 8, **characterized in that** the detection of the predetermined respiratory phase(s) is carried out on the basis of specified threshold values for the measurement values measured for determination of the respiratory phase, which measurement values must lie above or below the respective threshold values.

10. Method according to one of claims 7 to 9, **characterized in that** the respiratory phase is determined with the aid of a device for determining the respiratory phase (3)comprising at least one respiratory phase sensor (9), wherein the CO2 content, the humidity, the temperature, the flow and/or the pressure of the respiratory air flowing towards or away from a patient is measured with the aid of the at least one respiratory phase sensor (9).

11. Method according to one of claims 7 to 10, **characterized in that** the gas sensor (7) is used to measure the end-tidal oxygen content in the exhaled air of a patient.

## Revendications

1. Dispositif de mesure de la teneur en au moins un gaz dans l'air expiré (1), présentant au moins un dispositif de détermination de la phase respiratoire (3), au moins une unité de commande (4), au moins une soupape à plusieurs voies (6) commandable, présentant au moins une entrée et au moins deux sorties, et au moins un dispositif de pompage (5) ainsi qu'au moins un capteur de gaz (7), la phase respiratoire actuellement présente pouvant être déterminée dans un cycle respiratoire à l'aide du dispositif de détermination de la phase respiratoire (3), et la soupape commandable (6) et le dispositif de pompage (5) pouvant être commandés en fonction de la phase respiratoire détectée avec l'unité de commande (4), de telle sorte qu'un échantillon de gaz respiratoire de l'air respiratoire s'écoulant vers un patient ou s'éloignant d'un patient selon la phase respiratoire peut être guidé à travers le capteur de gaz (7) ou peut être isolé dans la zone du capteur de gaz (7), de telle sorte que la teneur d'un gaz dans l'air expiré peut être déterminée à l'aide du capteur de gaz (7) dans une section prédéterminée du cycle respiratoire, le dispositif de détermination de la phase respiratoire (3) présentant au moins un capteur de phase respiratoire (9) qui est réalisé sous la forme d'un capteur rapide avec un temps de montée inférieur à 500 ms, au moins un capteur de gaz (7) étant réalisé sous la forme d'un capteur d'O2 et le capteur d'O2 étant réalisé sous la forme d'un capteur lent avec un temps de montée supérieur à 500 ms pour déterminer la teneur en O2 dans un échantillon de gaz respiratoire et la soupape à plusieurs voies (6) commandable étant agencée dans la direction d'écoulement de l'échantillon de gaz respiratoire après le dispositif de pompage (5) et avant le capteur de gaz (7), une entrée de la soupape à plusieurs voies (6) étant reliée au dispositif de pompage (5), une sortie de la soupape à plusieurs voies (6) étant reliée au capteur de gaz (7) et une autre sortie de la soupape à plusieurs voies (6) étant reliée à une sortie (8), de telle sorte que l'échantillon de gaz respiratoire peut être conduit directement dans le capteur de gaz (7) ou vers la sortie (8), selon la position de soupape de la soupape à plusieurs voies (6) commandable.

2. Dispositif (1) selon la revendication 1, **caractérisé en ce que** le dispositif de détermination de la phase respiratoire (3) est réalisé pour mesurer dans le courant principal.

3. Dispositif (1) selon l'une quelconque des revendications 1 et 2, **caractérisé en ce que** le dispositif de détermination de la phase respiratoire (3) est réalisé pour mesurer dans le courant secondaire.

4. Dispositif (1) selon l'une quelconque des revendications 1 à 3, **caractérisé en ce qu'**au moins un capteur de phase respiratoire (9) est réalisé sous la forme d'un capteur de CO2, d'un capteur de température, d'un capteur d'humidité, d'un capteur de débit ou d'un capteur de pression.

5. Dispositif (1) selon l'une quelconque des revendications précédentes, **caractérisé en ce que** la soupape commandable (6) est réalisée sous la forme d'une soupape à plusieurs voies présentant au moins deux entrées et au moins deux sorties, de telle sorte qu'en fonction de la phase respiratoire, des échantillons de gaz respiratoire provenant de différents points du système peuvent être guidés à travers le capteur de gaz (7) ou peuvent être isolés dans la zone du capteur de gaz (7).

6. Dispositif de ventilation (20) présentant au moins un dispositif de mesure de la teneur en au moins un gaz dans l'air expiré (1) selon l'une quelconque des revendications précédentes.

7. Procédé de mesure de la teneur en au moins un gaz dans l'air expiré, utilisant un dispositif de mesure de la teneur en au moins un gaz dans l'air expiré (1) selon l'une quelconque des revendications 1 à 5, présentant au moins les étapes de procédé suivantes :
a. la détermination d'une phase respiratoire avec un capteur de phase respiratoire ayant un temps de montée inférieur à 500 ms
b. la détection de si une phase respiratoire prédéterminée est présente dans un cycle respiratoire
c. l'amenée d'un échantillon de gaz à au moins un capteur de gaz (7) réalisé sous la forme d'un capteur d'O2 avec un temps de montée supérieur à 500 ms
d. l'isolement de l'échantillon de gaz dans la zone de l'au moins un capteur de gaz (7)
e. la mesure de l'échantillon de gaz amené à l'aide de l'au moins un capteur de gaz (7)
f. la suppression de l'isolement de l'échantillon de gaz.

8. Procédé selon la revendication 7, **caractérisé en ce que** la détermination de la phase respiratoire et l'identification de si une phase respiratoire prédéterminée est présente sont effectuées en continu et en parallèle avec les autres étapes de procédé.

9. Procédé selon l'une quelconque des revendications 7 et 8, **caractérisé en ce que** l'identification de la ou des phases respiratoires prédéterminées s'effectue à partir de valeurs seuils prédéfinies pour les valeurs de mesure acquises pour la détermination de la phase respiratoire, qui doivent être respectivement dépassées vers le haut ou vers le bas.

10. Procédé selon l'une quelconque des revendications 7 à 9, **caractérisé en ce que** la phase respiratoire est effectuée à l'aide d'un dispositif de détermination de la phase respiratoire (3) présentant au moins un capteur de phase respiratoire (9) ; la teneur en CO2, l'humidité, la température, le débit et/ou la pression de l'air respiratoire s'écoulant vers un patient ou s'éloignant d'un patient étant mesurés à l'aide de l'au moins un capteur de phase respiratoire (9).

11. Procédé selon l'une quelconque des revendications 7 à 10, **caractérisé en ce qu'**à l'aide du capteur de gaz (7), on mesure la teneur finale en oxygène dans l'air expiré d'un patient.
